# EUROPEAN PATENT SPECIFICATION

(11) **EP 1 223 935 B1**
(45) Date of publication and mention of the grant of the patent: **09.01.2008**
(21) Application number: 00948537.6
(22) Date of filing: 29.06.2000
(51) Int. Cl.: A61K 31/47, A61P 31/00

(54) **USE OF GEMIFLOXACIN COMPOUNDS AGAINST BACTERIA**
VERWENDUNG VON GEMIFLOXACIN VERBINDUNGEN GEGEN BAKTERIEN
UTILISATION DE COMPOSES DE GEMIFLOXACINE CONTRE LES BACTERIES

(30) Priority: 29.06.1999 US 141416 P; 29.06.1999 US 141458 P; 29.06.1999 US 141409 P; 29.06.1999 US 141457 P; 29.06.1999 US 141455 P; 29.06.1999 US 141456 P; 29.06.1999 US 141487 P; 29.06.1999 US 141488 P; 02.07.1999 GB 9915597; 08.07.1999 US 142729 P; 08.07.1999 US 142725 P; 08.07.1999 US 142724 P; 14.09.1999 US 395492; 14.09.1999 US 395851; 21.09.1999 US 400144; 21.09.1999 US 399660; 21.09.1999 US 339661; 21.09.1999 US 399657; 21.09.1999 US 399855; 21.09.1999 US 399662; 22.09.1999 US 401433; 22.09.1999 US 401432; 12.05.2000 US 569648; 22.05.2000 US 577731; 22.05.2000 US 577732
(43) Date of publication of application: 24.07.2002
(73) Proprietor: LG Life Sciences, Ltd., Seoul 150-721 (KR)
(72) Inventor: APPELBAUM, Peter C., Hershey, PA 17033 (US); BAST, Darren, Hamilton, Ontario L8V 1C8 (CA); CITRON, Diane M., Santa Monica, CA 90404 (US); CRABB, Donna M., Birmingham, AL 35294 (US); CREDITO, Kim L., Hershey, PA 17033 (US); DAVIDSON, Ross J., Halifax, Nova Scotia B3H 2V8 (CA); DAVIES, Todd, Hershey, PA 17033 (US); DEAZAVEDO, Joyce, Toronto, Ontario M5G 1XG (CA); DUBOIS, Jacques, Fleurimont, G1J 4W2 (CA); DUFFY, Lynn B., Birmingham, AL 35294 (US); DUNCAN, Carla, Hamilton, Ontario L8S 4L8 (CA); ERNIE, Lois M., Hershey, PA 17033 (US); GOLDSTEIN, Ellie J.C., Santa Monica, CA 90404 (US); HOELLMAN, Dianne, B., Hershey, PA 17033 (US); KELLY, Linda M., Hershey, PA 17033 (US); LOW, Donald E., Toronto, Ontario M5G 1XG (CA); PANKUCH, Glenn A., Hershey, PA 17033 (US); SEARCEY, Karen B., Birmingham, AL 35294 (US); ST-PIERRE, Claude, St-Elie d'Orford, J0B 2S0 (CA)
(74) Representative: Gillard, Richard Edward
(86) International application number: PCT/US2000/017900
(87) International publication number: WO 2001/000209

(56) References cited:
- US-A- 4 931 446
- US-A- 5 756 506
- KIM ET AL: "Synthesis and antibacterial activities of LB20304: a new fluoronaphthyridone antibiotic containing novel oxime functionalized pyrrolidine" ANTIMICROBIAL AGENTS AND CHEMOTHERAPY, AMERICAN SOCIETY FOR MICROBIOLOGY, WASHINGTON, DC, US, 17 September 1995 (1995-09-17), page 148 XP002146747 ISSN: 0066-4804
- MORRISSEY I ET AL: "THE BACTERIAL ACTIVITY OF GEMIFLOXACIN AND OTHER FLUOROQUINOLONES AGAINST S. PNEUMONIAE" JOURNAL OF ANTIMICROBIAL CHEMOTHERAPY, SAUNDERS CO. LTD., LONDON, GB, vol. 44, no. SUPPL A, July 1999 (1999-07), page 147 XP000911924 ISSN: 0305-7453
- CORMICAN M G ET AL: "STUDIES TO ESTABLISH QUALITY CONTROL RANGES FOR SB-265805 (LB20304)WHEN USING NATIONAL COMMITTEE FOR CLINICAL LABORATORY STANDARDS ANTIMICROBIAL SUSCEPTIBILITY TEST METHODS" JOURNAL OF CLINICAL MICROBIOLOGY, WASHINGTON, DC, US, vol. 37, no. 1, January 1999 (1999-01), pages 279-280, XP000861920 ISSN: 0095-1137
- JOHNSON D M ET AL: "ANTI-STREPTOCOCCAL ACTIVITY OF SB-265805 (LB20304, A NOVEL FLUORONAPHTHYRIDONE, COMPARED WITH FIVE OTHER COMPOUNDS, INCLUDING QUALITY CONTROL GUIDELINES" DIAGNOSTIC MICROBIOLOGY AND INFECTIOUS DISEASES, ELSEVIER SCIENCE PUBLISHING CO., AMSTERDAM, NL, vol. 33, no. 2, February 1999 (1999-02), pages 87-91, XP000911956 ISSN: 0732-8893
- OH J-I ET AL: "IN VITRO AND IN VIVO EVALUATIONS OF LB20304, A NEW FLUORONAPHTHYRIDONE" ANTIMICROBIAL AGENTS AND CHEMOTHERAPY, AMERICAN SOCIETY FOR MICROBIOLOGY, WASHINGTON, DC, US, vol. 40, no. 6, June 1996 (1996-06), pages 1564-1568, XP000911979 ISSN: 0066-4804
- KIM M-Y ET AL: "IN VITRO ACTIVITIES OF LB20304, A NEW FLUOROQUINOLONE" ARCHIVES OF PHARMACAL RESEARCH, NATL. FISHERIES UNIVERSITY, PUSAN, KR, vol. 19, no. 1, 1996, pages 52-59, XP000911954 ISSN: 0253-6269
- CORMICAN M G ET AL: "ANTIMICROBIAL ACTIVITY AND SPECTRUM OF LB20304, A NOVEL FLUORONAPHTHYRIDONE" ANTIMICROBIAL AGENTS AND CHEMOTHERAPY, AMERICAN SOCIETY FOR MICROBIOLOGY, WASHINGTON, DC, US, vol. 41, no. 1, January 1997 (1997-01), pages 204-211, XP000911943 ISSN: 0066-4804
- OH J I ET AL: "IN VITRO AND IN VIVO ANTIBACTERIAL ACTIVITIES OF LB20304, A NEW FLUORONAPHTHYRIDONE" ANNUAL ICAAC ABSTRACTS, XX, XX, 12 September 1995 (1995-09-12), page 148 XP000861824
- KING A ET AL: "THE COMPARATIVE IN VITRO ACTIVITY OF GEMIFLOXACIN, A NEW FLUOROQUINOLONE, AGAINST SELECTED CLINICAL ISOLATES" JOURNAL OF ANTIMICROBIAL CHEMOTHERAPY, SAUNDERS CO. LTD., LONDON, GB, vol. 44, no. SUPPL A, 4 July 1999 (1999-07-04), page 147 XP000911926 ISSN: 0305-7453
- PAEK K-S ET AL: "BACTERICIDAL ACTIVITIES OF LB20304, A NEW FLUOROQUINOLONE" ARCHIVES OF PHARMACAL RESEARCH, NATL. FISHERIES UNIVERSITY, PUSAN, KR, vol. 19, no. 4, 1996, pages 317-320, XP000911955 ISSN: 0253-6269
- DUBOIS, J. AND ST-PIERRE, C.: "Comparative in vitro activity of gemifloxacin and other quinolones against maxillary sinus pathogens" JOURNAL OF ANTIMICROBIAL CHEMOTHERAPY, vol. 44, no. suppl. A, 4 July 1999 (1999-07-04), page 135 XP002271828
- MARCO F ET AL: "ANTIMICROBIAL ACTIVITY OF LB20304, A FLUORONAPHTHYRIDONE, TESTED AGAINST ANAEROBIC BACTERIA" JOURNAL OF ANTIMICROBIAL CHEMOTHERAPY, SAUNDERS CO. LTD., LONDON, GB, vol. 40, no. 4, 1997, pages 605-607, XP000911923 ISSN: 0305-7453
- DUBOIS, J. AND ST-PIERRE, C.: "Comparative in vitro activity and post-antibiotic effect of gemifloxacin against Legionella spp" JOURNAL OF ANTIMICROBIAL CHEMOTHERAPY, vol. 45, no. s1, April 2000 (2000-04), pages 41-46, XP002271832

## Description

### FIELD OF THE INVENTION

This invention relates, in part, to newly identified uses of a gemifloxacin compound against atypical upper respiratory pathogenic bacteria of the genus Legionella and Mycoplasma bacteria, such as *Mycoplasma pneumoniae.*

### BACKGROUND OF THE INVENTION

Quinolones have been shown to be effective to varying degrees against a range of bacterial pathogens. However, as diseases caused by these pathogens are on the rise, there exists a need for antimicrobial compounds that are more potent than the present group of quinolones.

Gemifloxacin mesylate (SB-265805) is a novel fluoroquinolone useful as a potent antibacterial agent. Gemifloxacin compounds are described in detail in patent application PCT/KR98/00051 published as WO 98/42705. Patent application EP 688772 discloses novel quinoline(naphthyridine)carboxylic acid derivatives, including anhydrous (R,S)-7-(3-aminomethyl-4-methoxyiminopyrrolidin-1-yl)-1-cyclopropyl-6-fluoro-4-oxo-1,4-dihydro-1,8-naphthyridine-3-carboxylic acid of formula I.

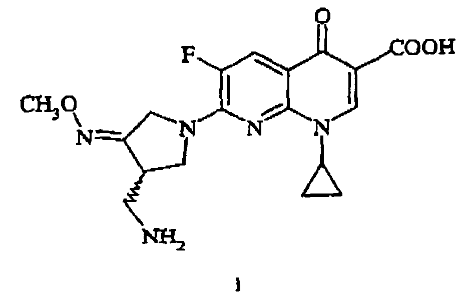

PCT/KR98/00051 discloses (R,S)-7-(3-aminomethyl-4-*syn*-methoxyimino-pyrrolidin-1-yl)-1-cyclopropyl-6-fluoro-4-oxo-1,4-dihydro-1,8-naphthyridine-3-carboxylic acid methanesulfonate and hydrates thereof including the sesquihydrate.

Provided herein is an invention based, in part, on a number of significant discoveries made using a gemifloxacin compound against a range of bacteria.

While *in vitro* testing of new antimicrobial compounds is often extensive, these studies tend to focus on a limited range of typical anaerobic bacterial pathogens (Cormicon, et al., Antimicrob. Agents Chemother., 41:204-211, 1997; Hohl, et al., Clin. Microbiol. Infect., 4:280-284, 1998; Marco, et al., J. Antimicrob. Chemother., 40:605-607, 1997). Moreover, the activity of gemifloxacin against typical anaerobic bacteria has been reported (Goldstein, et al., *Antimicrob. Agents Chemother.,* Submitted); it showed activity against *Bacteroides fragilis* and certain *Prevotella* and *Porphyromonas* strains, but only limited activity against *B*. *thetalotaomicron, B. distasonis* and *B. ovatus.* Data is lacking about the activities of new quinolone compounds against many of the less frequently encountered anaerobic pathogens, such as *Actinomyces* spp., *Anaerobiospirrilum* spp., *Porphyromonas* spp., and *Bilophila wadsworthia.*

Provided herein is an invention based, in part, on a significant discovery made using a gemifloxacin compound against a range of variety of *Legionella* isolated from nosocomial or acquired respiratory tract infections and from environmental sources, demonstrating the activity of the gemifloxacin compound used was superior to a number of quinolones as described in more detail herein. Gemifloxacin compounds are valuable compounds for the treatment of diseases caused by or related to atypical respiratory tract pathogens, thereby filling still another unmet medical need.

Moreover, the instant invention provides a significant discovery made using a gemifloxacin compound against *Mycoplasma,* demonstrating the activity of the gemifloxacin compound used was superior to a number of quinolones as described in more detail herein. Gemifloxacin compounds are valuable compounds for the treatment of bacterial infection caused by a range of *Mycoplasma* pathogens, including those resistant to usual oral therapy, thereby filling another unmet medical need.

KIM et al. in "Synthesis and antibacterial activities of LB20304: a new fluoronaphthyridone antibiotic containing novel oxime functionalized pyrrolidine" ANTIMICROBIAL AGENTS AND CHEMOTHERAPY, AMERICAN SOCIETY FOR MICROBIOLOGY, WASHINGTON, DC, US, 17 September 1995, page 148 discloses the antibacterial activity of gemifloxacin (LB20304) against several pathogens including S. aureus, P. aeruginosa, E. faecalis.

MORRISSEY I et al. in "THE BACTERIAL ACTIVITY OF GEMIFLOXACIN AND OTHER FLUOROQUINOLONES AGAINST S. PNEUMONIAE" JOURNAL OF ANTIMICROBIAL CHEMOTHERAPY, SAUNDERS CO. LTD., LONDON, GB, vol. 44, no. SUPPL A, July 1999, page 147 discloses the antibacterial activity of gemifloxacin (LB20304) against S. pneumoniae, including ciprofloxacin-resistant strains.

CORMICAN M G et al. in "STUDIES TO ESTABLISH QUALITY CONTROL RANGES FOR SB-265805 (LB20304) WHEN USING NATIONAL COMMITTEE FOR CLINICAL LABORATORY STANDARDS ANTIMICROBIAL SUSCEPTIBILITY TEST METHODS" JOURNAL OF CLINICAL MICROBIOLOGY, WASHINGTON, DC, US, vol. 37, no. 1, January 1999, pages 279-280 discloses the antibacterial activity of gemifloxacin (LB20304) against several pathogens, including S. aureus, P. aeruginosa, E. faecalis, S. pneumoniae and H. influenzae.

JOHNSON D M et al. in "ANTI-STREPTOCOCCAL ACTIVITY OF SB-265805 (LB20304), A NOVEL FLUORONAPHTHYRIDONE, COMPARED WITH FIVE OTHER COMPOUNDS, INCLUDING QUALITY CONTROL GUIDELINES" DIAGNOSTIC MICROBIOLOGY AND INFECTIOUS DISEASES, ELSEVIER SCIENCE PUBLISHING CO., AMSTERDAM, NL, vol. 33, no. 2, February 1999, pages 87-91 discloses the antibacterial activity of gemifloxacin (LB20304/SB265805) against S. pneumoniae, including ciprofloxacin-resistant strains.

OH J-I et al. in "IN VITRO AND IN VIVO EVALUATIONS OF LB20304, A NEW FLUORONAPHTHYRIDONE" ANTIMICROBIAL AGENTS AND CHEMOTHERAPY, AMERICAN SOCIETY FOR MICROBIOLOGY, WASHINGTON, DC, US, vol. 40, no. 6, June 1996, pages 1564-1568 discloses the antibacterial activity of gemifloxacin (LB20304) against several pathogens, including S. pneumoniae and P. aeruginosa.

KIM M-Y et al. in "IN VITRO ACTIVITIES OF LB20304, A NEW FLUOROQUINOLONE" ARCHIVES OF PHARMACAL RESEARCH, NATL. FISHERIES UIVIVERSTTY, PUSAN, KR, vol. 19, no. 1, 1996, pages 52-59 discloses the antibacterial activity of gemifloxacin (LB20304) against several pathogens, including S. aureus, P. aeruginosa, S. pneumoniae.

CORMICAN M G et al. in "ANTIMICROBIAL ACTIVITY AND SPECTRUM OF LB20304, A NOVEL FLUORONAPHTHYRIDONE" ANTIMICROBIAL AGENTS AND CHEMOTHERAPY, AMERICAN SOCIETY FOR MICROBIOLOGY, WASHINGTON, DC, US, vol. 41, no. 1, January 1997, pages 204-211 discloses the antibacterial activity of gemifloxacin (LB20304) against several pathogens, including S. aureus, P. aeruginosa, S. pneumoniae, Clostridium, Peptostreptococcus and H. influenzae.

OH J 1 et al. in "IN VITRO AND IN VIVO ANTIBACTERIAL ACTIVITIES OF LB20304, A NEW FLUORONAPHTHYRIDONE" ANNUAL ICAAC ABSTRACTS, XX, XX, 12 September 1995, page 148, and PAEK K-S et al. in "BACTERICIDAL ACTIVITIES OF LB20304, A NEW FLUOROQUINOLONE" ARCHIVES OF PHARMACAL RESEARCH, NATL. FISHERIES UNIVERSITY, PUSAN, KR, vol. 19, no. 4, 1996, pages 317-320 discloses the antibacterial activity of gemifloxacin (LB20304) against S. aureus and P. aeruginosa.

MARCO F et al. in "ANTIMICROBIAL ACTIVITY OF LB20304, A PLUORDNAPHTHYRIDONE, TESTED AGAINST ANAEROBIC BACTERIA" JOURNAL OF ANTIMICROBIAL CHEMOTHERAPY, SAUNDERS CO. LTD., LONDON, GB, vol. 40, no. 4, 1997, pages 605-607, XP000911923 ISSN: 0305-7453 discloses the antibacterial activity of gemifloxacin (LB20304) against anaerobic bacteria, in particular Peptostreptococcus.

An object of the invention is the use of gemifloxacin compound in the preparation of a medicament for modulating metabolism of atypical upper respiratory pathogenic bacteria comprising the step of contacting atypical upper respiratory pathogenic bacteria with an antibacterially effective amount of said composition, comprising a germifloxacin-compound, or an antibacterially effective derivative thereof.

The said atypical upper respiratory pathogenic bacteria are selected from the group consisting of: a member of the genus *Legionella,* a *Legionella pneumophila* strain, a *Legionella pneumophila* serogroup 1, a *Legionella pneumophila* serogroup 2, a *Legionella pneumophila* serogoup 3, a *Legionella pneumophila* serogroup 4, a *Legionella pneumophila* serogroup 5, a *Legionella pneumophila* serogroup 6, a *Legionella pneumophila* serogroup 7, a *Legionella pneumophila* serogroup 8, a *Legionella dumoffii* strain, a *Legionella longbeacheae* strain, a *Legionella micdadei* strain, a *Legionella oakridgensis* strain, a *Legionella feelei* strain, a *Legionella anisa* strain, a *Legionella sainthelensi* strain, a *Legionella bozemanii* strain, a *Legionella wadsworthii* strain, a *Legionella jordanis* strain and a *Legionella gormanii* strain.

Also provided by the invention is the use of a gemifloxacin compound in the preparation of a medicament for treating or preventing a bacterial infection by atypical upper respiratory pathogenic bacteria comprising the step of administering an antibacterially effective amount of a composition comprising a gemifloxacin compound to a mammal suspected of having or being at risk of having an infection with atypical upper respiratory pathogenic bacteria.

Further preferred embodiments are provided by the invention wherein said bacteria of the genus *Legionella* is. a *L. pneumophila* strain, a *L pneumophila* serogroup 1, a *L pneumophila* serogroup 2, a *L pneumophila* serogroup 3, a *L. pneumophila* serogroup 4, a *L pneumophila* serogroup 5, a *L. pneumophila* serogroup 6, a *L pneumophila* serogroup 7, a *L. pneumophila* serogroup 8, a *L durnoffii* strain, a *L longbeacheae* strain, a L. miedadei strain, a L oakridgensis strain, a *L. feelei* strain, a *L. anisa* strain, a *L sainthelensi* strain, a *L bozemanii s*train, a *L*. *wadsworthii* strain, a *L. jordanis* strain and a *L. gormanii* strain.

It is preferred in the uses of the invention that said contacting is performed once daily.

An object of the invention is the use of a gemifloxacin compound in the preparation of a medicament for modulating metabolism of pathogenic Mycoplasma bacteria comprising the step of contacting pathogenic Mycoplasma bacteria with an antibacterially effective amount of a composition comprising a gemifloxacin compound, or an antibacterially effective derivative thereof.

A further object of the invention is the use wherein said pathogenic Mycoplasma bacteria is selected from the group consisting of: *Mycoplasma pneumoniae, M. hominis, M. fermentans, M. genitalium, M. penetrans* and *Ureaplasma urealyticum.*

Also provided by the invention is the use of a gemifloxacin compound in the preparation of a medicament for treating or preventing a bacterial infection by pathogenie Mycoplasma bacteria comprising the step of administering an antibacterially effective amount of a composition comprising a gemifloxacin compound to a mammal suspected of having or being at risk of having an infection with pathogenic Mycoplasma bacteria.

Further preferred uses are provided by the invention wherein said bacteria is selected from the group consisting of: *Mycoplasma pneumoniae, M. hominis, M. fermentants, M. genitalium, M. penetrans* and *Ureaplasma urealyticum.*

A preferred use of the invention uses a gemifloxacin compound that is a gemifloxacin mesylate, particularly hydrates, especially hemihydrates and sesquihydrates thererof.

Another preferred use is provided wherein said modulating metabolism is inhibiting growth of said bacteria or killing said bacteria.

A further preferred use is provided wherein said contacting said bacteria comprises the further step of introducing said composition into a mammal, particularly a human.

A use is also provided using a gemifloxacin compound in veterinary applications particularly in livestock.

### DESCRIPTION OF THE INVENTION

### (A) Methods of Using Fluoroquinolones Against Atypical Upper Respiratory Tract Bacteria

The present invention provides, among other things, methods for using a composition comprising a gemifloxacin compound against atypical upper respiratory pathogenic bacteria, especially a member of the genus *Legionella,* a *L*. *pneumophila* strain. a *L. pneumophila* serogroup 1. a *L. pneumophila* serogroup 2, a *L pneumophila* serogroup 3. a *L pneumophila* serogroup 4. a *L pneumophila* serogroup 5, a *L. pneumophila* serogroup 6, a *L. pneumophila* serogroup 7. a *L. pneumophila* serogroup 8, a *L. durnoffii* strain, a *L. longbeacheae* strain, a L. micdadei strain. a L. oakridgensis strain, a *L*. *feelei* strain, a *L. anisa* strain, a *L. sainthelensi* strain, a *L. bozemanii* strain, a *L. wodsworthii* strain, a *L. jordanis* strain or a *L. gormanii* strain.

As used herein "gemifloxacin compound(s)" means a compound having antibacterial activity described in patent application PCT/KR98/00051 published as WO 98/42705. or patent application EP 688772.

This invention was based, in part, on analyses evaluating the *in vitro* activity and postantibiotic effect (herein "PAE") of gemifloxacin compared with those of trovafloxacin, moxifloxacin, grepafloxacin, levofloxacin, ofloxacin, ciprofloxacin, azithromycin, clarithromycin, erythromycin and rifampicin against isolates of *Legionella pneumophila* and other *Legionella* spp. Test isolates included *L. pneumophila* serogroup 1-12 (204), *L*. *dutmoffii* (10), *L micdadei* (10) and *L. longbeacheae* (7). The PAE was determined by exposing the isolates to the test antimicrobials for 1 hour at four times the minimum inhibitory concentration (herein "MIC"). The antimicrobial was removed by three consecutive centrifugations into fresh broth. The PAE was calculated by measuring bacterial growth kinetics in similar antimicrobial-free cultures. Rifampicin and trovafloxacin were the most active agents tested (MiC₉₀ ≤0.008 mg/L). Gemifloxacin displayed high potency (MIC₅₀ 0.016 mg/L) which was comparable to levofloxacin, grepafloxacin and moxifloxacin (MIC₉₀ 0.016 mg/L) and more active than ciprofloxacin and ofloxacin (MIC₉₀ 0.03 mg/L). Against *L. dumoffii* and *L. longheachae,* gemifloxacin (MIC₉₀ 0.06 mg/L) was as active than ciprofloxacin, ofloxacin, grepafloxacin and moxifloxacin. Against erythromycin-resistant *L. pneumophila,* gemifloxacin showed the longest PAE at 4.65 hours, compared with 4.18 hours for grepafloxacin, 3.38 hours for moxifloxacin and 2.83 hours for trovafloxacin. The gemifloxacin PAE was significantly superior to that of rifampicin (0.9 h), clarithromycin (1.9 h) and levofloxacin (2.59 h). Against erythromycin-susceptible *L. pneumophila* only gemifloxacin, moxifloxacin, levofloxacin, ofloxacin and ciprofloxacin had a PAE over 3 hours- For erythromycin-resistant *Legionella* spp. other than *L. pneumophila,* gemifloxacin, moxifloxacin, levofloxacin and ofloxacin had PAEs in excess of 3 hour, which was significantly longer than the PAE of ciprofloxacin grepafloxacin and erythromycin. The half-life for gemifloxacin and the data presented here indicate a significant PAE to support a once-daily administration of this agent for the treatment of *Legionella* infections, and this dosing is preferred in the methods of the invention.

The MIC range of gemifloxacin against *L pneumophila* serogroups 1-9 was 0.008-0.06 mg/L (Tables 2 and 3). Gemifloxacin was 5-6-fold more active than erythromycin against *L*. *pneumophila* strains tested. Gemifloxacin activity against *L. pneumophila* strains was higher than ciprofloxacin and ofloxacin but similar to grepafloxacin and moxifloxacin.

*L. pneumophila* strains of sera groups 1-3 and 7-9 were more susceptible to gemifloxacin than *L. pneumophila* serogroups 4-6. Against the most frequent *L. pneumophila,* such as serogroup 1, gemifloxacin MIC₉₀ (0.016 mg/L) was superior to azithromycin, clarithromycin, erythromycin, ofloxacin and ciprofloxacin.

Against *L. dumoffii* and *L. longbeacheae,* gemifloxacin, grepafloxacin and clarithromycin showed superior activity to azithromycin and erythromycin (Table 4). Against *L micdadei,* gemifloxacin, ciprofloxacin, ofloxacin and moxifloxacin MICs were 5-fold more active than erythromycin.

Against mythromycin-resistant *L. pneumophila* only gemifloxacin, moxifloxacin and grepafloxacin displayed a mean PAE of >3 hours (Table 5 ). Clarithromycin, erythromycin and rifampicin showed a PAE of <2 hours against these strains- Against erythromycin-resistant *Legionella* spp. other than *pneumophila,* gemifloxacin, grepafloxacin, levofloxacin, ofloxacin and rifampicin displayed a mean PAE of >3 hours, and erythromycin and clarithromycin of <2 hours.

Against erythromycin-susceptible *L. pneumophila,* gemifloxacin, moxifloxacin, ofloxacin and ciprofloxacin displayed a mean PAE of >3hours. Gemifloxacin and ofloxacin were the only quinolones showing a mean PAE of >2 hours against erythromycin-susceptible *Legionella* spp. other than *L pneumophila.*

Gemifloxacin is an effective antimicrobial agent against most *Legionella* spp. and was significantly superior to the commonly used legionellosis therapy, erythromycin.

Against erythromycin-susceptible *L pneumophila,* the mean PAE of gemifloxacin (3.49 hours) was ≥1 h longer than that of trovafloxacin, levofloxacin and clarithromycin.

Against erythromycin-susceptible *Legionella* spp. other than *pneumophila* the mean PAE of gemifloxacin (2.27 hours) was ≥1 h longer than that of trovafloxacin, moxifloxacin and clarithromycin.

Against erythromycin-resistant *L. pneumophila,* the mean PAE of gemifloxacin (4.65 hours) was significantly superior to the mean PAE of trovafloxacin, levofloxacin, ciprofloxacin, azithromycin, clarithromycin, erythromycin and rifampicin. A difference in mean PAE was also noted between gemifloxacin and trovafloxacin against *Legionella* spp. other than *L. pneumophila.*

The results of this study indicate that gemifloxacin should be a promising agent for the treatment of lower respiratory tract infections caused by *Legionella* spp.

**Table L. Legionella Strains Tested**

| Microrganism | No. of strains tested |
|---|---|
| *L. pneumophila* | 204* |
| *L. micdadei* | 10 |
| *L. dumoffii* | 10 |
| *L. longbeacheae* | 7 |
| Others^{†} | 7 |

| | |
|---|---|
| * 10 different serogroups ^{†} *oakridgensis, feelei, anisa, sainthelensi, bozemanii, gormanii*, *wadsworthii* | |

**Table 2 Susceptibility of Logionella pneumophila Serogroups 1-4**

| Antimicrobial | *L. pneumophila* serogroup 1 (n=85) | | | *L-pneumophila* serogroup 2 (n=17) | | | *L-pneumophila* serogroup 3 (n=15) | | | *L. pneumophila* serogroup 4 (n=26) | | |
|---|---|---|---|---|---|---|---|---|---|---|---|---|
| | MIC (mg/L) | | | MIC (mg/L) | | | MIC (mg/L) | | | MIC(mg/L) | | |
| | Range | 50% | 90% | Range | 50% | 90% | Range | 50% | 90% | Range | 50% | 90% |
| Gemifloxacin | 0.008-0.06 | 0.016 | ≤0.004 | 0.008-0.016 | 0.008 | 0.016 | 0.008-0.416 | 0.016 | 0.016 | 0.008-0.03 | 0.016 | 0.03 |
| Trovafloxacin | ≤0.004-0.016 | ≤0.004 | ≤0.004 | ≤0.004 | ≤0.004 | ≤0.004 | ≤0.004 | ≤0.004 | ≤0.004 | ≤0.004 | ≤0.004 | ≤0.004 |
| Moxifloxacin | ≤0.004-0.03 | 0.016 | 0.016 | ≤04.004-0.016 | 0.008 | 0.008 | ≤0.004-0.016 | 0.008 | 0.016 | ≤0.004-0.016 | 0.016 | 0.016 |
| Grepafloxacin | ≤0.004-0.06 | 0.016 | 0.016 | ≤0.004-0.03 | 0.008 | 0.016 | ≤0.004-0.016 | 0.008 | 0.016 | 0.008-0.016 | 0.008 | 0.016 |
| Lavofloxacin | ≤0.004-0.016 | 0.016 | 0.016 | ≤0.004-0.016 | 0.008 | 0.008 | 0.008-0.016 | 0.008 | 0.016 | 0.004-0.016 | 0.016 | 0.016 |
| Ofloxacin | 0.008-0.03 | 0.03 | 0.03 | 0.008-0.03 | 0.016 | 0.03 | 0.016-0.03 | 0.016 | 0.03 | 0.008-0.03 | 0.03 | 0.03 |
| Ciprofloxacin | 0.016-0.25 | 0.03 | 0.03 | ≤0.004-0.03 | 0.016 | 0.016 | ≤0.004-0.03 | 0.03 | 0.03 | 0.016-0.12 | 0.03 | 0.06 |
| Axithromycin | 0.008-1.0 | 0.06 | 0.5 | 0.008-0.12 | 0.06 | 0.12 | 0.016-0.25 | 0.12 | 0.25 | 0.008-0.25 | 0.12 | 0.12 |
| Clarithromycin | ≤0.004-0.12 | 0.06 | 0.06 | ≤0.004-0.06 | 0.03 | 0.06 | 0.016-0.06 | 0.03 | 0.06 | 0.004-0.06 | 0.03 | 0.06 |
| Erythromycin | 0.03-1.0 | 0.25 | 1.0 | 0.008-0.5 | 0.25 | 0.25 | 0.06-0.5 | 0.25 | 0.5 | 0.016-0.5 | 0.5 | 0.5 |
| Rifampicin | ≤0.004-0.008 | ≤0.004 | 0.008 | ≤0.004 | ≤0.004 | ≤0.004 | ≤4.004 | ≤0.004 | ≤0.004 | ≤0.004-0.008 | ≤0.004 | ≤0.004 |

**Table 3. Susceptibility of L, pneumophila Serogroups 5-12**

| Antimicrobial | *L. pneumophila* serogroup 5 (n = 15) | | | *L. pneumophila* serogroup 6 (n = 40) | | | *L. pneumophila* serogroup 7 (n=2) | | | *L. pneumophila* serogroups 8, 9 and 12 (n = 4) | | |
|---|---|---|---|---|---|---|---|---|---|---|---|---|
| | MIC (mg/L) | | | MIC (mg/L) | | | MIC (mg/L) | | | MIC (mg/L) | | |
| | Range | 50% | 90% | Range | 50% | 90% | Range | 50% | 90% | Range | 50% | 90% |
| Gemifloxacin | 0.03-006 | 0.03 | 0.03 | 0.008-0.03 | 0.016 | 0.03 | 0.008-0.016 | 0.008 | 0.016 | 0.016 | 0.016 | 0.016 |
| Trovafloxacin | ≤0.004-0.008 | ≤0.004 | ≤0.004 | ≤0.004 | ≤0.004 | ≤0.004 | ≤0.004 | ≤0.004 | ≤0.004 | ≤0.004 | ≤0.004 | ≤0.004 |
| Moxifloxacin | ≤0.004-0.03 | 0.016 | 0.016 | ≤0.004-0.016 | 0.008 | 0.016 | ≤0.004-0.016 | ≤0.004 | 0.016 | 0.016 | 0.016 | 0.016 |
| Grepafloxacin | ≤0.004-0.003 | 0.016 | 0.03 | ≤0.004-0.016 | 0.008 | 0.016 | ≤0.004-0.008 | ≤0.004 | 0.008 | 0.008 | 0.008 | 0.008, |
| Levofloxacin | ≤0.004-0.016. | 0.008 | 0.016 | 0.008-0.016 | 0.008 | 0.016 | 0.008-0.016 | 0.008 | 0.016 | 0.008-0.016 | 0.008 | 0.016 |
| Ofloxacin | 0.008-0.03 | 0.016 | 0.03 | 0.008-0.03 | 0.03 | 0.03 | 0.03 | 0.03 | 0.03 | 0.03 | 0.03 | 0.03 |
| Ciprofloxacin | 0.016-0.06 | 0.03 | 0.03 | ≤0.004-0.03 | 0.03 | 0.03 | 0,03 | 0.03 | 0.03 | 0.03 | 0.03 | 0.03 |
| Azithromycin | 0.008-0.5 | 0.03 | 0.25 | 0.016-0.25 | 0.06 | 0.12 | 0,06 | 0.06 | 0.06 | 0.06 | 0,06 | 0.06 |
| Clarithromycin | 0.03-0.06 | Q.03 | 0.06 | ≤0.004-0.06 | 0.016 | 0.06 | 0.016-0.06 | 0.016 | 0.06 | 0.06 | 0.06 | 0.06 |
| Erythromycin | 0.06-1.0 | 0.25 | 0.5 | 0.008-0.25 | 0.12 | 0.25 | 0.12-0.5 | 0.12 | 0.5 | 0.25 | 0.25 | 0.25 |
| Rifamplelal | ≤0.004 | ≤0.004 | ≤0.004 | ≤0.004-0.008 | ≤0.004 | ≤0.004 | **≤**0.004 | ≤0.004 | ≤0.004 | ≤0.004 | ≤0.004 | ≤0.004 |

**Table 4. Susceptibility of Legionella Other Than pneumophila**

| Antimicrobial | *L dumoffii* (o = 10) | | | *L micdadel* (n = 10) | | | *L longbeachene* (n = 7) | | | Other *Legionella* spp. (n = 7)* | | |
|---|---|---|---|---|---|---|---|---|---|---|---|---|
| | MIC (mg/L) | | | MIC (mg/L) | | | MIC (mg/L) | | | MIC (mg/L) | | |
| | Range | 50% | 90% | Range | 50% | 90% | Range | 50% | 90% | Range | 50% | 90% |
| Gemifloxacln | 0.06 | 0.06 | 0.06 | 0.008-0.03 | 0.016 | 0.03 | 0.016-0.06 | 0.06 | 0.06 | 0.016-0.06 | 0.03 | 0.06 |
| Trovafloxacin | 50.004- 0.008 | 0.008 | 0.008 | ≤0.004 | ≤0.004 | ≤0.004 | ≤0.004 | ≤0.004 | ≤0.004 | ≤0.004 | ≤0.004 | ≤0.004 |
| Moxifloxacin | 0.008- 0.03 | 0.03 | 0.03 | 0.008-0.03 | 0.016 | 0.03 | 0.008-0.03 | 0.016 | 0.03 | 0.008-0.03 | 0.008 | 0.03 |
| Grepafloxacin | 0.06 | 0.06 | 0.06 | ≤0.004-0.016 | 0.008 | 0.016 | ≤0.004-0.06 | 0.03 | 0.46 | ≤0.004-0.03 | 0.03 | 0.03 |
| Levofloxacin | 0.016 | 0.016 | 0.016 | 0.008-0.016 | 0.016 | 0.016 | 0.008-0.016 | 0.016 | 0.016 | 0.008-0.06 | 0.016 | 0.016 |
| Ofloxacin | 0.03 | 0.03 | 0.03 | 0.03 | 0.03 | 0.03 | 0.016-0.03 | 0.03 | 0.03 | ≤0.004-0.06 | 0.016 | 0.06 |
| Ciprofloxacin | 0.016- 0.03 | 0.016 | 0.03 | 0.016-0.03 | 0.016 | 0.03 | ≤0,004-0.03 | 0,016 | 0.03 | ≤0.004-0.03 | 0.016 | 0.03 |
| Azithromycin | 0.12-0.25 | 0.12 | 0.25 | 0.016-0.25 | 0.25 | 0.25 | 0.016-0.23 | 0.12 | 0.25 | 0.016-0.5 | 0.12 | 0.5 |
| Clarithromycin | 0.03-0,06 | 0.03 | 0.06 | 0.03-0,12 | 0.06 | 0.06 | 0.008-0.06 | 0.06 | 0.06 | ≤0.004-0.12 | 0.03 | 0.12 |
| Erythromycin | 0.25-0.5 | 0.25 | 0.5 | 0.25-1 | 0.5 | 1 | 0.008-0.5 | 0.25 | 0.5 | 0.016-1 | 0.5 | 1 |
| Rifampicin | ≤0.004- 0.03 | 0.008 | 0.016 | 0.008 | 0.008 | 0.008 | ≤0.004-0.06 | ≤0.004 | 0.06 | ≤0.004-0.008 | ≤0.004 | 0.008 |

| | | | | | | | | | | | | |
|---|---|---|---|---|---|---|---|---|---|---|---|---|
| *Includes one isolates of L *bozemanii, L. feelei, L. jordanis. L. garmanii, L oakridgensis, L. sainthelensi* and *L wadsworthii,* | | | | | | | | | | | | |

**Table 5. Mean PAE of Antimicrobials Against Erythromycin-resistant and susceptible Strains of Legionella**

| **Antimicrobial (4xMIC)** | **Mean PAE (h)*** | | | |
|---|---|---|---|---|
| | **Erythromycin-resistant strains** | | **Erythromycin-susceptible strains** | |
| | ***L. pneumophila*** (n=7) | ***Legionella* spp.†** (n=9) | ***L. pneumophila*** (n=15) | ***Legionella* spp.**** (n=13) |
| **Gemifloxacin** | 4.63±3 | 3.34±2 | 3.49±3 | 2.27±2 |
| **Trovafloxacin** | 2.83 ±2 | 2.25±2 | 1.71±1 | 1.22±1 |
| **Moxifloxacin** | 3.38±2 | 2.02±1 | 3.59±3 | 1.18±2 |
| **Grepafloxacin** | 4.18±3 | 3.67±1 | 2.62±3 | 1.67±1 |
| **Levofloxacin** | 2.59±2 | 3.24±1 | 2.14±2 | 1.35±1 |
| **Ofloxacin** | 2.99±1 | 4.13±2 | 3.53±3 | 3.04±2 |
| **Ciprofloxacin** | 2.86±2 | 2.13±3 | 3.61±2 | 1.86±2 |
| **Azithromycin** | 2.16±1 | 2.13±1 | 2.91±3 | 1.86±2 |
| **Clarithromycin** | 1.90±1 | 1.60±2 | 0.72±2 | 0.98±2 |
| **Erythromycin** | 0.90±1 | 0.44±1 | 0.93±1 | 2.06±2 |
| **Rifampicin** | 0.93±4 | 5.6±3 | 2.86±5 | 3.09±4 |

| | | | | |
|---|---|---|---|---|
| *Means are given ± SD *†L. micdadei* (n=1), *L. dumofii* (n=3), *L. bozemanii* (n=1), *L*. *wadsworthii* (n=1), *L. Jordanis* (n=1), *L. longbeacheae* (n=2) ***L*. *micdadei* (n=4), *L damofii* (n=5), *L. bozemanii* (n=1), *L. gormanii* (n=1), *L. Jordanis* (n=1), *L. longbeacheae* (n=1) | | | | |

The invention provides a use for modulating metabolism of atypical upper respiratory pathogenic bacteria as described above. Skilled artisans can readily choose atypical upper respiratory pathogenic bacteria or patients infected with or suspected to be infected with these organisms to practice the methods of the invention, whereby the bacteria useful in the methods of the invention, are those described herein.

Also provided by the invention is a use of a gemifloxacin compound in the preparation of a medicament for treating or preventing a bacterial infection by atypical upper respiratory pathogenic bacteria comprising the step of administering an antibacterially effective amount of a composition comprising a gemifloxacin compound to a mammal, preferably a human, suspected of having or being at risk of having an infection with atypical upper respiratory pathogenic bacteria as described above.

A preferred object of the invention provides a method wherein said atypical upper respiratory pathogenic bacteria is selected from the group consisting of: a member of the genus *Legionella,* a *L. pneumophila* strain, a *L. pneumophila* serogroup 1, a *L. pneumophila* serogroup 2, *a L pneumophila* serogroup 3. a *L. pneumophila* serogroup 4. a *L. pneumophila* serogroup 5, a *L. pneumophila* serogroup 6, a L *pneumophila* serogroup 7, a *L*. *pneumophila* serogroup 8. a *L. dumoffii* strain, a L *longbeacheae* strain, a L. micdadei strain, a L. oakridgensis strain, a *L. feelei* strain, a *L. anisa* strain, a *L. sainthelensi* strain, a *L. bozemanii* strain, a *L wadsworthii* strain, a *L. jordanis:* strain and a *L. gormanii* strain. The skilled artisan may identify these organisms as provided herein as well as using other methods known in the art. *e.g.* MIC tests.

### (B) Methods of Using Fluoroquinolones Against Mycoplasma Bacteria

The present invention provides, among other things, methods for using a composition comprising a gemifloxacin compound against a range of pathogenic bacteria.

This invention was based, in part, on analyses evaluating the *in vitro* activity of a gemifloxacin compound, as well as other new quinolones and macrolides using low-passaged clinical isolates and type strains of *Mycoplasma* species commonly found in the respiratory and urogenital tract of humans. Organisms used in the analyses included *Mycoplasma pneumoniae* (MPN), *M. hominis* (Mh), *M. fermentans* (*Mf*), *M. genitalium* (Mg), *M. penetrans* (Mp) and *Ureaplasma urealyticum* (Uu). Minimum Inhibitory Concentrations (MICs) were determined using a micro-broth dilution method. Assays for *Ureaplasma urealyticum* were performed in 10B, media and all other mycoplasma assays were carried out in SP4 medium. Comparator drugs, to which gemifloxacin was compared, as well as also being useful in the methods of the invention, include levofloxacin (Lev), trovafloxacin (Tro), grepafloxacin (Gre), azithromycin (Azi), clarithromycin (Cla), tetracycline (Tet) and clindamycin (Cli)- The results of these MIC assays are shown in Table 6.

**TABLE 6**

| **MIC 90 (ug/ml)** | | | | | | | | |
|---|---|---|---|---|---|---|---|---|
| **Isolates (number)** | **Gem** | **Lev** | **Trov** | **Grep** | **Azith** | **Clar** | **Tet** | **Clin** |
| MPN(103) | 0.125 | 0.5 | 0.25 | 0.125 | ≤0.008 | ≤0.008 | 0.25 | - |
| Mh(49) | ≤0.008 | 0.25 | 0.031 | 0.031 | - | - | 32 | ≤0.008 |
| Mf(19) | ≤0.008 | 0.031 | 0.016 | 0.016 | 2 | 64 | 0.063 | 0.031 |
| Un(99) | 0.25 | 1 | 0.125 | 1 | 4 | 0.063 | 1 | - |
| MICs for Mg (2) | 0.063 | 1 | 0.063 | 0.125 | ≤0.008 | ≤0.008 | 0.125 | 0.25 |
| | 0.063 | 0.5 | 0.063 | 0.125 | ≤0.008 | ≤0.008 | 0.063 | 0.25 |
| MICs for Mp (1) | ≤0.008 | 0.031 | ≤0.008 | 0.016 | ≤0.008 | ≤0.008 | 0.125 | ≤0.008 |

Depending on the species tested, gemifloxacin had variable results when compared to the macrolides. Gemifloxacin was equally as active or more active in *vitro* when compared to tetracycline, clindamycin and the other quinolones.

The invention provides a USE for modulating metabolism of pathogenic Mycoplasma bacteria. Skilled artisans can readily choose pathogenic Mycoplasma bacteria or patients infected with or suspected to be infected with these organisms to practice the methods of the invention. Alternatively, the bacteria useful in the methods of the invention are those described herein.

Also provided by the invention is a use of a gemifloxacin compound in the preparation of a medicament for treating or preventing a bacterial infection by pathogenic Mycoplasma bacteria comprising the step of administering an antibacterially effective amount of a composition comprising a gemifloxacin compound to a mammal, preferably a human, suspected of having or being at risk of having an infection with pathogenic Mycoplasma bacteria.

While a preferred object of the invention provides a method wherein said pathogenic Mycoplasma bacteria is selected from the group consisting of: *Mycoplasma pneumoniae, M. hominis, M. fermentans, M. genitalium, M. penetrans* and *Ureaplasma urealyticum.* Other pathogenic Mycoplasma bacteria may also be included in the methods. The skilled artisan may identify these organisms as provided herein as well as using other methods known in the art. *e.g*. MIC tests.

The contacting step in any of the uses of the invention may be performed in many ways that will be readily apparent to the skilled artisan. However, it is preferred that the contacting step is a provision of a composition comprising a gemifloxacin compound to a human patient in need of such composition or directly to bacteria in culture medium or buffer.

For example, when contacting a human patient or contacting said bacteria in a human patient or in *vitro,* particularly in any of the methods of the invention, the compositions comprising a gemifloxacin compound, preferably pharmaceutical compositions may be administered in any effective, convenient manner including, for instance, administration by topical, oral, anal, vaginal, intravenous, intraperitoneal, intramuscular, subcutaneous, intranasal or intradermal routes among others.

It is also preferred that these compositions be employed, in any of the uses of the invention, in combination with a non-sterile or sterile carrier or carriers for use with cells, tissues or organisms, such as a pharmaceutical carrier suitable for administration to a subject. Such compositions comprise, for instance, a media additive or a therapeutically effective amount of a compound of the invention, a gemifloxacin compound, and a pharmaceutically acceptable carrier or excipient. Such carriers may include, but are not limited to saline, buffered saline, dextrose, water, glycerol, ethanol and combinations thereof. The formulation should suit the mode of administration. Gemifloxacin compounds and compositions of the uses of the invention may be employed, in any of the uses of the invention, alone or in conjunction with other compounds, such as bacterial efflux pump inhibitor compounds or antibiotic compounds, particularly non-quinolone compounds, *e.g.,* beta-lactam antibiotic compounds.

In therapy or as a prophylactic, the active agent of a method of the invention is preferably administered to an individual as an injectable composition, for example as a sterile aqueous dispersion, preferably an isotonic one.

Alternatively, the gemifloxacin compounds or compositions in any of the uses of the invention may be formulated for topical application for example in the form of ointments, creams, lotions, eye ointments, eye drops, ear drops, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

For administration to mammals, and particularly humans, it is expected that the antibacterially effective amount is a daily dosage level of the active agent from 0.001 mg/kg to 10 mg/kg, typically around 0.1 mg/kg to 1 mg/kg, preferably about 1 mg/kg. A physician, in any event, will determine an actual dosage that is most suitable for an individual and will vary with the age, weight and response of the particular individual. The above dosages are exemplary of the average case. There can, of course, be individual instances where higher or lower dosage ranges are merited, and such are within the scope of this invention. It is preferred that the dosage is selected to modulate metabolism of the bacteria in such a way as to inhibit or stop growth of said bacteria or by killing said bacteria. The skilled artisan may identify this amount as provided herein as well as using other methods known in the art, *e.g.* by the application MIC tests.

A further embodiment of the invention provides for the contacting step of the uses to further comprise contacting an in-dwelling device in a patient. In-dwelling devices include, but are not limited to, surgical implants, prosthetic devices and catheters. i.e., devices that are introduced to the body of an individual and remain in position for an extended time. Such devices include, for example, artificial joints, heart valves, pacemakers, vascular grafts, vascular catheters, cerebrospinal fluid shunts, urinary catheters, and continuous ambulatory peritoneal dialysis (CAPD) catheters.

A gemifloxacin compound or composition of the invention may be administered by injection to achieve a systemic effect against a bacteria of the invention, shortly before insertion of an in-dwelling device. Treatment may be continued after surgery during the in-body time of the device. In addition, the composition could also be used to broaden perioperative cover for any surgical technique to prevent bacterial wound infections.

In addition to the therapy described above, a gemifloxacin compound or composition used in the methods of this invention may be used generally as a wound treatment agent to prevent adhesion of bacteria to matrix proteins, exposed in wound tissue and for prophylactic use in dental treatment as an alternative to, or in conjunction with, antibiotic prophylaxis.

Alternatively, a gemifloxacin compound or composition of the invention may be used to bathe an indwelling device immediately before insertion. The active agent will preferably be present at a concentration of 1µg/ml to 10mg/ml for bathing of wounds or indwelling devices.

Preferred embodiments of the invention include, among other things, uses wherein said composition comprises gemifloxacin, or a pharmaceutically acceptable derivative thereof. EXAMPLES

The present invention is further described by the following examples. The examples are provided solely to illustrate the invention by reference to specific embodiments.

All examples were carried out using standard techniques, which are well known and routine to those of skill in the art, except where otherwise described in detail.

All parts or amounts set out in the following examples are by weight, unless otherwise specified.

### Example 1 Bacterial Strains

A variety of *Legionella* were isolated from respiratory tract and environmental sources. Identification of organisms was by standard methods known in the art (for example, see, Washington, C.W. Jr. Legionella. In: Murray et al., eds. Manual of Clinical Microbiology. 6th ed. American Society of Microbiology 1993: 533-544).

### Example 2 Susceptibility Testing

MICs for *Legionella* strains were determined by standard 2-fold agar dilution procedure using Buffered Yeast Extract agar (herein "BYE") (National Committee for Clinical Laboratory Standards: Methods for antimicrobial susceptibility tests for bacteria that growth aerobically, approved standards M 7-A4. National Committee for Laboratory Standards, Villanova, PA. 1997).

A final innoculum of about 10⁴ colony forming units (herein "CFU") was inoculated onto the BYE containing doubling dilutions of antibiotics (0.004-256 mg/L). Plates were incubated at 35°C for 48 hours. An MIC was defined as the lowest concentration of antimicrobial that completely inhibited visible growth. Strains of *Pseudomonas aeruginosa* ATCC 27853 and *L*. *pneumophila* ATCC 33152 were included as controls.

### Example 3 Determination of PAE

The *in vitro* method using the broth technique (Craig, W-A- Antibiotics in laboratory medicine. Williams & Wilkins 1986: 515-536) was used to determine the PAE with Buffered Yeast extract (BYE). Each strain of Example 1̅ was exposed to antimicrobial concentration of four times the MIC. Fresh inoculum (1 ml, final concentration of 10⁶-10⁷ CFU/ml) was added to 9 ml of prepared antimicrobial containing medium and to 9 ml of drug-free control medium and incubated at 37°C for 1-2 hours. Antimicrobial agent was removed by three consecutive centrifugations at 1200 x g for 10 minutes. Counts of CFU/ml were performed on all cultures at time zero, before and after washing, and every 1 hour until turbidity develops. The counts of CFU/ml were graphed and the duration of PAE was calculated by equation:

PAE=T·C

where T is the time required for the count of CFU in the test culture to increase 1 log₁₀ above the count observed immediately after drug removal, and C is the time required for the count of untreated control culture to increase by 1 log₁₀ above the count observed immediately after the completion of the same procedure used on the test culture for drug removal.

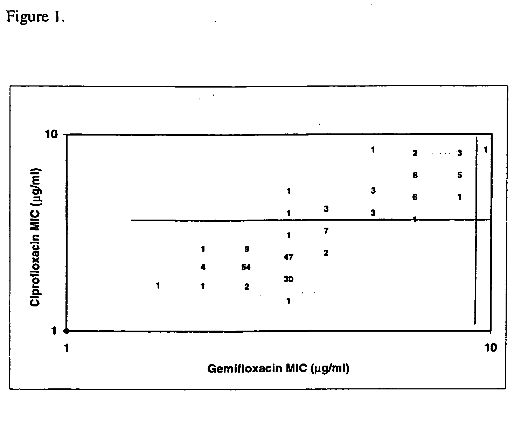

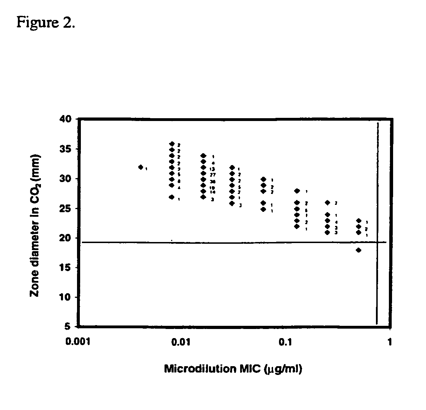

## Claims

1. Use of gemifloxacin or a pharmaceutically acceptable salt thereof in the making of an antibacterially effective medicament composition for treating or preventing a bacterial infection by pathogenic Mycoplasma bacteria in a mammal suspected of having or being at risk of having an infection with pathogenic Mycoplasma bacteria.

2. Use of gemifloxacin or a pharmaceutically acceptable salt thereof in the making of an antibacterially effective medicament composition for killing or inhibiting the growth of pathogenic Mycoplasma bacteria in a mammal.

3. The use as claimed in claim 1 or 2, wherein said pathogenic Mycoplasma bacteria arc selected from the group consisting of: *Mycoplasma pneumoniae, M. hominis, M. fermentans, M. genitalium, M. penetrans* and *Ureaplasma urealyticum.*

4. Use of gemifloxacin or a pharmaceutically acceptable salt thereof in the making of an antibacterially effective medicament composition for treating or preventing a bacterial infection by atypical upper respiratory pathogenic bacteria in a mammal suspected of having or being at risk of having an infection with atypical upper respiratory pathogenic bacteria,
wherein the bacteria are a member of the genus *Legionella*.

5. Use of gemifloxacin or a pharmaceutically acceptable salt thereof in the making of an antibacterially effective medicament composition for killing or inhibiting the growth of atypical upper respiratory pathogenic bacteria in a mammal,
wherein the bacteria are a member of the genus *Legionella.*

6. The use as claimed in claim 4 or 5 wherein said bacteria are of a *Legionella pneumophila* strain.

7. The use as claimed in claim 6 wherein said bacteria are selected from the group consisting of a *L. pneumophila* serogroup 1, a *L. pneumophila* serogroup 2, a *L. pneumophila* serogroup 3, a *L*. *pneumophila* serogroup 4, a *L. pneumophila* serogroup 5, a. *L*. *pneumophila* serogroup 6, a *L. pneumophila* serogroup 7, and a *L. pneumophila* serogroup 8.

8. The use as claimed in claim 4 or 5 wherein said bacteria are selected from the group consisting of: a *L. dumoffii* stram, a *L. longbeacheae* strain, a *L. micdadei* strain, a *L. oakridgensis* strain, a *L. feelei* strain, a *L. anisa* strain, a *L. sainthelensi* strain, a *L*. *bozemanii* strain, *a L. wadsworthii* strain, a *L. jordanis* strain, and a *L. gormanii* strain.

9. The use as claimed in claim 4 or 5 wherein the medicament composition is for administering to the mammal once daily.

10. The use as claimed in any one of the preceding claims wherein said mammal is a human.

11. The use as claimed in any one of the preceding claims, wherein the gemifloxacin or the pharmaceutically acceptable salt thereof is gemifloxacin mesylate or a hydrate thereof.

12. The use as claimed in claim 11 wherein the gemifloxacin mesylate or the hydrate thereof is gemifloxacin mesylate sesquihydrate.

## Patentansprüche

1. Verwendung von Gemifloxacin oder eines pharmazeutisch annehmbaren Salzes davon zur Herstellung einer antibakteriell wirksamen Arzneimittelzusammensetzung zur Behandlung oder Verhütung einer bakteriellen Infektion durch pathogene Mycoplasma-Bakterien bei einem Säuger, bei dem der Verdacht oder die Gefahr besteht, eine Infektion durch ein pathogenes Mycoplasma-Bakterium zu haben.

2. Verwendung von Gemifloxacin oder eines pharmazeutisch annehmbaren Salzes davon zur Herstellung einer antibakteriell wirksamen Arzneimittelzusammensetzung zum Abtöten oder zur Wachstumshemmung von pathogenen Mycoplasma-Bakterien bei einem Säuger.

3. Verwendung nach Anspruch 1 oder 2, worin das pathogene Mycoplasma-Bakterium aus der Gruppe bestehend aus *Mycoplasma pneumoniae, M. hominis, M. fermentans, M. genitalium, M. penetrans und Ureaplasma urealyticum* ausgewählt ist.

4. Verwendung von Gemifloxacin oder eines pharmazeutisch annehmbaren Salzes davon zur Herstellung einer antibakteriell wirksamen Arzneimittelzusammensetzung zur Behandlung oder Verhütung einer bakteriellen Infektion durch atypische, für die oberen Atemwege pathogene Bakterien bei einem Säuger, bei dem der Verdacht oder die Gefahr besteht, eine Infektion durch atypische, für die oberen Atemwege pathogene Bakterien zu haben, worin die Bakterien zur Gattung *Legionella* gehören.

5. Verwendung von Gemifloxacin oder eines pharmazeutisch annehmbaren Salzes davon zur Herstellung einer antibakteriell wirksamen Arzneimittelzusammensetzung zum Abtöten und zur Wachstumshemmung von atypischen, für die oberen Atemwege bei einem Säuger pathogenen Bakterien, worin die Bakterien zur Gattung *Legionella* gehören.

6. Verwendung nach Anspruch 4 oder 5, worin die Bakterien von einem *Legionella pneumophila-*Stamm abstammen.

7. Verwendung nach Anspruch 6, worin die Bakterien aus der Gruppe bestehend aus *L. pneumophila* Serogruppe 1, *L. pneumophila* Serogruppe 2, *L. pneumophila* Serogruppe 3, *L. pneumophila* Serogruppe 4, *L. pneumophila* Serogruppe 5, *L. pneumophila* Serogruppe 6, *L. pneumophila* Serogruppe 7 und *L. pneumophila* Serogruppe 8 ausgewählt sind.

8. Verwendung nach Anspruch 4 oder 5, worin die Bakterien aus der Gruppe bestehend aus einem *L. dumoffii-*Stamm, einem *L. longbeacheae-*Stamm, einem *L. micdadei-*Stamm, einem *L. oakridgensis-*Stamm, einem *L. feelei-*Stamm, einem *L.* anisa-Stamm, einem *L. sainthelensi-*Stamm, einem *L. bozemanii-*Stamm, einem *L. wadsworthii-*Stamm, einem *L. jordanis-*Stamm und einem *L. gonnanii-*Stamm ausgewählt sind.

9. Verwendung nach Anspruch 4 oder 5, worin die Arzneimittelzusammensetzung zur einmal täglichen Verabreichung an einem Säuger bestimmt ist.

10. Verwendung nach einem der vorangehenden Ansprüche, worin der Säuger ein Mensch ist.

11. Verwendung nach einem der vorangehenden Ansprüche, worin das Gemifloxacin oder das pharmazeutisch annehmbare Salz davon Gemifloxacinmesylat oder ein Hydrat davon ist.

12. Verwendung nach Anspruch 11, worin das Gemifloxacinmesylat oder das Hydrat davon Gemifloxacinmesylatsesquihydrat ist.

## Revendications

1. Utilisation de gémifloxacine ou d'un sel pharmaceutiquement acceptable de celle-ci dans la préparation d'une composition médicamenteuse antibactérienne efficace pour traiter ou prévenir une infection bactérienne par des bactéries Mycoplasma pathogènes chez un mammifère suspecté, ou à risque, d'infection par des bactéries Mycoplasma pathogènes.

2. Utilisation de gémifloxacine ou d'un sel pharmaceutiquement acceptable de celle-ci dans la préparation d'une composition médicamenteuse antibactérienne efficace pour tuer ou inhiber la croissance de bactéries Mycoplasma pathogènes chez un mammifère.

3. Utilisation selon la revendication 1 ou 2, dans laquelle lesdites bactéries Mycoplasma pathogènes sont choisies dans le groupe constitué de : *Mycoplasma pneumoniae, M*. *hominis, M*. *fermentans, M*. *genitalium, M*. *penetrans* et *Ureaplasma urealyticum.*

4. Utilisation de gémifloxacine ou d'un sel pharmaceutiquement acceptable de celle-ci dans la préparation d'une composition médicamenteuse antibactérienne efficace pour traiter ou prévenir une infection bactérienne par des bactéries pathogènes des voies respiratoires aériennes atypiques chez un mammifère suspecté, ou à risque, d'infection par des bactéries pathogènes des voies respiratoires aériennes atypiques, dans laquelle les bactéries appartiennent au genre *Legionella.*

5. Utilisation de gémifloxacine ou d'un sel pharmaceutiquement acceptable de celle-ci dans la préparation d'une composition médicamenteuse antibactérienne efficace pour tuer ou inhiber la croissance de bactéries pathogènes des voies respiratoires aériennes atypiques chez un mammifère, dans laquelle les bactéries appartiennent au genre *Legionella.*

6. Utilisation selon la revendication 4 ou 5, dans laquelle lesdites bactéries proviennent d'une souche de *Legionella pneumophila.*

7. Utilisation selon la revendication 6, dans laquelle lesdites bactéries sont choisies dans le groupe constitué de : un sérogroupe 1 de *L. pneumophila,* un sérogroupe 2 de *L. pneumophila,* un sérogroupe 3 de *L. pneumophila,* un sérogroupe 4 de *L. pneumophila,* un sérogroupe 5 de *L. pneumophila,* un sérogroupe 6 de *L. pneumophila,* un sérogroupe 7 de *L. pneumophila,* et un sérogroupe 8 de *L. pneumophila.*

8. Utilisation selon la revendication 4 ou 5, dans laquelle lesdites bactéries sont choisies dans le groupe constitué de : une souche de *L. dumoffii,* une souche de *L. longbeacheae,* une souche de *L*. *micdadei,* une souche de *L. oakridgensis,* une souche de *L. feelei,* une souche de *L.* anisa, une souche de *L. sainthelensi,* une souche de *L*. *bozemanii,* une souche de *L. wadsworthii,* une souche de *L. jordanis,* et une souche de *L*. *gormanii.*

9. Utilisation selon la revendication 4 ou 5, dans laquelle la composition médicamenteuse est à administrer à un mammifère à raison d'une fois par jour.

10. Utilisation selon l'une quelconque des revendications précédentes, dans laquelle ledit mammifère est l'homme.

11. Utilisation selon l'une quelconque des revendications précédentes, dans laquelle la gémifloxacine ou le sel acceptable de celle-ci est un mésylate de gémifloxacine ou un hydrate de celui-ci.

12. Utilisation selon la revendication 11, dans laquelle le mésylate de gémifloxacine ou son hydrate est le sesquihydrate de mésylate de gémifloxacine.
